# FASCICULE DE BREVET EUROPEEN

(11) **EP 1 361 827 B1**
(45) Date de publication et mention de la délivrance du brevet: **08.08.2007**
(21) Numéro de dépôt: 02704879.2
(22) Date de dépôt: 22.02.2002
(51) Int. Cl.: A61B 17/86

(54) **VIS DE FIXATION**
BEFESTIGUNGSSCHRAUBE
FIXING SCREW

(30) Priorité: 22.02.2001 FR 0102372
(43) Date de publication de la demande: 19.11.2003
(73) Titulaire: Abbott Spine, La Cité Mondiale, 33000 Bordeaux (FR)
(72) Inventeur: MAZDA, Keyvan, F-75020 Paris (FR); LE COUEDIC, Régis, 33000 Bordeaux (FR)
(74) Mandataire: Dronne, Guy
(86) Numéro de dépôt international: PCT/FR2002/000658
(87) Numéro de publication internationale: WO 2002/065929

(56) Documents cités:
- EP-A- 0 230 856
- DE-A- 2 019 040
- DE-A- 19 720 782
- DE-A- 19 943 594
- US-A- 2 247 499
- US-A- 3 842 824
- US-B1- 6 176 659

## Description

La présente invention concerne une vis de fixation comportant un corps longitudinal fileté s'étendant entre une tête destinée à l'entraîner en rotation et une extrémité formant pointe susceptible d'être insérée dans un support.

De tels vis sont connues par exemple du document EP-A-0230856, et le préambule de la revendication 1 est basé sur cet art antérieur.

Un domaine d'application envisagé est notamment, mais non exclusivement celui des dispositifs de stabilisation du rachis pour lesquels il est nécessaire d'ancrer des fixations dans les vertèbres en insérant, dans les pédicules, des vis longitudinales qui se prolongent dans le corps vertébral.

Les dispositifs de stabilisation du rachis permettent, en cas de traumatisme ou de pathologies dégénératives du rachis de fixer ou de guider le déplacement des vertèbres, les unes par rapport aux autres, afin de préserver l'intégrité de la moelle épinière. Pour ce faire, des éléments de fixation sont reliés aux vertèbres par des vis, dites vis pédiculaires, et les éléments de fixation des vertèbres superposées sont reliés entre eux par des tiges de liaison qui assurent la rigidité du rachis.

Les vis, dont le corps longitudinal est fileté de la tête jusqu'à l'extrémité formant pointe selon un pas relativement large sont insérées de façon oblique depuis la paroi arrière du rachis dans le pédicule de la vertèbre jusque dans le corps vertébral. Généralement, deux vis sont insérées dans les deux pédicules des vertèbres de façon à fixer deux fixations de chaque côté de l'axe du rachis pour obtenir une stabilisation symétrique.

Cependant, les dimensions du squelette des individus sur lesquels des dispositifs de stabilisation du rachis sont susceptibles d'être installés, sont variables et par conséquent la section des vertèbres l'est aussi. Ainsi, la longueur des vis pédiculaires nécessaire à l'ancrage des moyens de fixation doit être choisie au cours de l'intervention par l'opérateur qui dispose d'une panoplie de vis de longueurs différentes.

Il a été imaginé de rendre sécable le corps longitudinal des vis afin d'adapter leurs longueurs à l'utilisation que l'on souhaite leur conférer. Un problème qui se pose et que vise à résoudre la présente invention, est alors de permettre l'introduction de la nouvelle extrémité de vis ainsi obtenue, dans le support.

Le US 2,247,499 décrit une tige-vis. La tige est constituée par une pluralité de vis, chaque vis étant raccordée aux vis adjacentes par des portions sécables. On peut ainsi obtenir une pluralité de vis, chaque vis comportant sa propre tête.

Le document DE-A-2019040 décrit une vis dont une portion est destinée à être retirée grâce à un point de rupture qui peut être formé d'une gorge pratiquée dans sa périphérie, après son insertion à travers des parties à joindre, pour éviter un bout projétant nuisible. Sa rupture ne doit donc pas former une nouvelle extrémité formant pointe.

Dans le but de résoudre le problème, la présente invention propose une vis dont le corps longitudinal fileté comprend : au moins un point de rupture pour permettre de rompre à force ledit corps longitudinal fileté au droit dudit point de rupture de façon à retirer une portion dudit corps longitudinal s'étendant dudit point de rupture à ladite extrémité formant pointe, ledit point de rupture étant formé d'une gorge pratiquée dans la périphérie dudit corps longitudinal fileté ; au moins une portion tronconique évasée vers la tête, ladite portion tronconique s'étendant dudit point de rupture vers ladite tête ; pour former une nouvelle extrémité formant pointe après que ladite portion dudit corps longitudinal a été retirée.

Ainsi, une caractéristique de la vis conforme à l'invention réside dans ses points de rupture qui, situés à des endroits déterminés sur le corps longitudinal fileté, permettent non seulement d'ajuster la longueur dudit corps aux dimensions de la vertèbre dans laquelle elle est vissée, mais aussi de l'introduire sans difficulté. De la sorte, d'une part il n'est pas nécessaire de disposer d'une batterie de vis de longueurs différentes, mais de sectionner une vis de longueur standard à la longueur désirée ; et d'autre part, la portion tronconique forme la nouvelle extrémité formant pointe, ce qui facilite le vissage. Bien évidemment, la longueur standard des vis est choisie de telle façon qu'elle soit inférieure à toutes les longueurs maximales possibles, et la position du point de rupture est choisie de manière à ce que la longueur du corps fileté une fois rompu, soit supérieure à toutes les longueurs minimales. En outre, le corps longitudinal fileté présente un point de rupture pour diminuer les efforts à lui appliquer pour le rompre. Pour cela, on peut utiliser un dispositif mécanique adapté pour rendre l'opération plus aisée, les pinces coupantes usuelles peuvent convenir.

En outre, le point de rupture est réalisé par un affaiblissement mécanique du corps longitudinal fileté produit par un évidement circulaire. Bien évidemment, la gorge qui est pratiquée dans le corps longitudinal est suffisamment ténue pour que la vis puisse être insérée en rotation dans les vertèbres sans rupture mécanique, et elle est suffisamment marquée pour permettre de rompre aisément le corps longitudinal fileté.

Selon un mode particulier de mise en oeuvre de l'invention, la paroi de la gorge la plus proche de ladite tête forme ladite portion tronconique, de façon à former une nouvelle extrémité formant pointe après que ledit corps longitudinal fileté a été rompu. Ainsi, selon cette caractéristique, non seulement la longueur du corps longitudinal fileté est susceptible d'être réduite mais l'extrémité de la vis est susceptible de former une pointe qui facilite le vissage à force dans la vertèbre.

De façon particulièrement avantageuse, ledit corps longitudinal fileté comprend un premier point de rupture et un deuxième point de rupture compris entre ladite tête et ledit premier point de rupture pour permettre de rompre à force ledit corps longitudinal fileté au droit dudit premier point de rupture de façon à retirer une première portion dudit corps longitudinal fileté s'étendant dudit premier point de rupture à ladite extrémité formant pointe, ou au droit dudit deuxième point de rupture de façon à retirer une deuxième portion dudit corps longitudinal fileté s'étendant dudit deuxième point de rupture à ladite extrémité formant pointe.

De la sorte, la vis conforme à l'invention est susceptible de présenter une longueur intermédiaire entre une longueur maximale et une longueur minimale. Cette caractéristique permet de mieux adapter la longueur de la vis aux dimensions de la vertèbre dans laquelle elle est introduite pour obtenir un ancrage suffisant et solidariser l'élément de fixation à la vertèbre.

Préférentiellement, ladite tête de vis forme une rotule dans laquelle est pratiqué un évidement, opposé audit corps longitudinal fileté, ledit évidement étant susceptible de recevoir une clé pour entraîner en rotation ladite tête. Ainsi, l'élément de fixation est relié avec des moyens appropriés à la rotule autour duquel il est partiellement articulé avant un serrage qui immobilise ledit élément par rapport à la rotule.

Selon une caractéristique avantageuse, la vis de fixation conforme à l'invention est réalisée en acier inoxydable. De façon préférentielle, elle est réalisée en alliage de titane qui est parfaitement bio-compatible et qui présente de bonnes performances mécaniques.

Selon un autre objet, la présente invention propose un ensemble de stabilisation du rachis, comprenant au moins une vis de fixation conforme à la présente invention, ladite vis de fixation étant susceptible d'être vissée dans le pédicule d'une vertèbre.

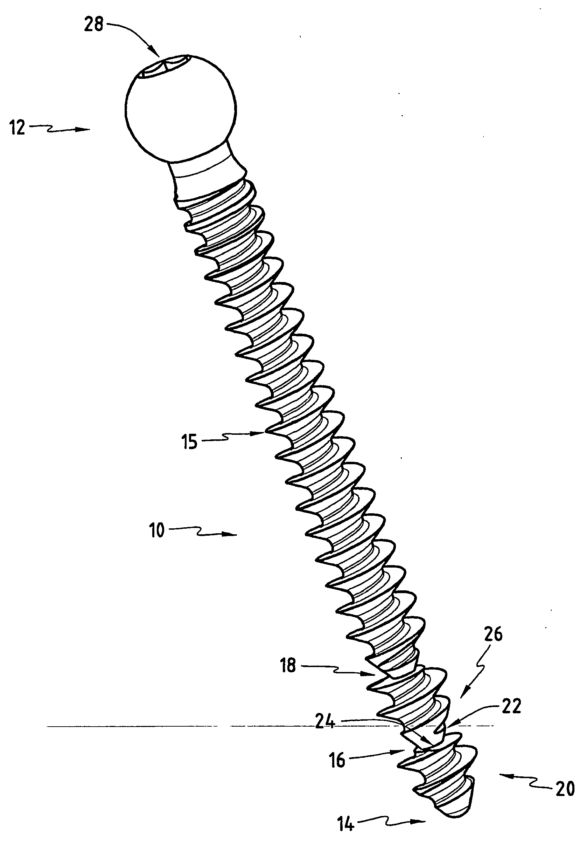

D'autres particularités et avantages de l'invention ressortiront à la lecture de la description faite ci-après de modes de réalisation particuliers de l'invention, donnés à titre indicatif mais non limitatif, en référence au dessin annexé sur lequel :
- la Figure unique est une vue en perspective de la vis de fixation, objet de l'invention, selon un mode particulier de réalisation.

La vis de fixation qu'illustre la Figure unique, comporte un corps longitudinal fileté 10 muni d'une tête 12 de symétrie sphérique et terminé par une extrémité formant pointe 14. Le corps longitudinal 10 est fileté sur toute sa longueur et comporte un seul filet, selon une configuration particulière et un pas adapté au matériau osseux dans lequel on insère la vis de fixation.

Le profil du filetage est dissymétrique, en dents de scie de façon que la composante axiale de l'action de contact du matériau dans lequel on insère la vis soit supérieure à la composante radiale. De la sorte, les filets 15 constituent des points d'arrêt efficaces contre l'arrachement de la vis selon son axe longitudinal.

L'extrémité formant pointe 14 du corps longitudinal 10, quoique légèrement arrondie, est suffisamment aiguë pour que l'application de ladite extrémité 14 avec un effort modéré contre le matériau osseux et l'entraînement en rotation de la vis produisent son introduction dans ledit matériau.

Une caractéristique essentielle de la vis de fixation selon l'invention réside dans le ménagement de points de rupture 16, 18, dans le corps longitudinal fileté 10 ; un premier point de rupture 16 est situé à proximité de l'extrémité formant pointe 14, définissant une portion 20 dudit corps longitudinal 10, et un deuxième point de rupture 18, est compris entre la tête 12 de vis et le premier point de rupture 16.

Les points de ruptures 16 et 18 sont réalisés en diminuant la section du corps longitudinal fileté 10 et plus précisément, comme l'illustre la Figure unique, en pratiquant une rainure circulaire à la périphérie du corps longitudinal. Cette rainure, ou gorge, présente deux parois opposées, inclinées l'une par rapport à l'autre et se rejoignant dans le font de la rainure. La paroi de la gorge 22 correspondant au point de rupture 16, qui forme un angle inférieur à 90° avec l'axe de la vis de fixation, est évasée vers la tête 12 de façon à former un tronc de cône. En revanche, la paroi opposée à la paroi de la gorge 22 est, elle, sensiblement perpendiculaire à l'axe de la vis de fixation. Une configuration identique est adoptée pour l'autre point de rupture 18.

L'intérêt de ces points de rupture 16 et 18, résident, bien évidemment, dans la possibilité de rompre le corps longitudinal 10 de la vis, selon l'un ou l'autre de ces points 16 ou 18, de façon à adapter la longueur de la vis à l'application à laquelle on la destine. Outre les dimensions du squelette qui détermine les dimensions de la section des vertèbres et donc la distance qui doit séparer les deux extrémités d'une vis pédiculaire, les vis de fixation, conforment à l'invention sont susceptibles d'être utilisées pour d'autres applications où l'insertion de la vis est réalisée depuis la partie antérieure du rachis directement dans le corps vertébral, et pour lesquelles la longueur des vis est différente. Ainsi, en réalisant une vis de fixation dont le corps longitudinal fileté est suffisamment long pour couvrir toutes les longueurs maximales possibles dans les différents types d'intervention et en ménageant au moins deux points de rupture suffisamment éloignés l'un de l'autre et de l'extrémité formant pointe, il est possible d'obtenir une vis dont la longueur est adaptée à toutes les situations.

En outre, l'invention n'est nullement limitée à un corps longitudinal dans lequel ne sont ménagées que deux points de rupture, mais elle concerne également une vis de fixation dans le corps longitudinal de laquelle on aurait pratiqué trois rainures pour adapter de façon plus appropriée la longueur de la vis de fixation.

Grâce aux points de ruptures 16, 18, formés par des rainures, le corps longitudinal fileté 10 est susceptible d'être raccourci à une longueur appropriée au moment de l'utilisation. Pour ce faire, un dispositif spécial, ou une pince coupante du commerce sont utilisés de façon à entraîner, par exemple, la portion 20 dans une direction sensiblement perpendiculaire à l'axe de la vis de fixation, cette dernière étant maintenue en position fixe, et à casser le corps longitudinal 10 au regard du point de rupture 16. En utilisant une pince coupante, les deux mors de la pince permettent de couper, de manière plus aisée, le corps longitudinal au regard du point de rupture où il y a moins de matière.

En outre, grâce à la forme évasée de la paroi de la gorge 22 correspondant au point de rupture 16, de façon à former un tronc de cône, lorsque la portion 20 est retirée, la rupture se situant sensiblement dans le font de la rainure ou les deux parois se rejoignent, elle laisse apparaître une nouvelle extrémité 24 formant pointe. Cette nouvelle extrémité formant pointe ne nécessite aucun usinage particulier et est susceptible de permettre l'introduction de la vis dans le matériau osseux.

Bien évidemment, lorsque l'on souhaite réduire encore la longueur du corps longitudinal 10, on procède de façon identique à celle qui est décrite ci-dessus, au niveau du point de rupture 18 situé entre la tête 12 et le point de rupture 16 et on retire la portion 26 de corps longitudinal.

Les vis de fixation conformes à l'invention sont réalisées en alliages de titane ou en acier inox, ou en tout autre matériau présentant les mêmes propriétés, et il va de soi qu'elles conservent leur résistance mécanique, non seulement pour la fixation mais aussi pour l'insertion à force dans le matériau osseux, lorsqu'elles comportent leurs rainures et qu'elles ne sont donc pas sectionnées.

L'invention concerne également un ensemble de stabilisation du rachis, non représenté et comprenant au moins une vis dont le corps longitudinal fileté comprend au moins un point de rupture pour permettre de rompre à force ledit corps longitudinal fileté au droit dudit point de rupture de façon à retirer une portion dudit corps longitudinal s'étendant dudit point de rupture à ladite extrémité formant pointe. Selon une caractéristique avantageuse, la tête de la vis forme une rotule autour de laquelle ledit dispositif de fixation est susceptible d'être articulé et sur laquelle il peut également être bloqué.

Avantageusement, la tête présente alors un évidement 28 central opposé au corps longitudinal fileté 10, susceptible de recevoir une clé pour entraîner la vis en rotation et l'introduire dans le matériau osseux.

## Revendications

1. Vis de fixation comportant un corps longitudinal fileté (10) s'étendant entre une tête (12) destinée à l'entraîner en rotation et une extrémité formant pointe (14) susceptible d'être insérée dans un support, **caractérisée en ce que** ledit corps longitudinal fileté (10) comprend :
- au moins un point de rupture (16) pour permettre de rompre à force ledit corps longitudinal fileté (10) au droit dudit point de rupture (16) de façon à retirer une portion (20) dudit corps longitudinal (10) s'étendant dudit point de rupture (16) à ladite extrémité formant pointe (14), ledit point de rupture (16) étant formé d'une gorge pratiquée dans la périphérie dudit corps longitudinal fileté (10), et
- au moins une portion tronconique évasée vers la tête (12), ladite portion tronconique s'étendant dudit point de rupture vers la tête (12) ; pour former une nouvelle extrémité formant pointe après que ladite portion (20) dudit corps longitudinale (10) a été retirée.

2. Vis de fixation selon la revendication 1, **caractérisée en ce que** la paroi de la gorge (22) la plus proche de ladite tête (12) forme ladite portion tronconique évasée vers ladite tête (12), de façon à former ladite nouvelle extrémité formant pointe (24) après que ledit corps longitudinal fileté (10) ait été rompu.

3. Vis de fixation selon l'une quelconque des revendications 1 et 2, **caractérisée en ce que** ledit corps longitudinal fileté comprend un premier point de rupture (16) et un deuxième point de rupture (18) compris entre ladite tête (12) et ledit premier point de rupture (16) pour permettre de rompre à force ledit corps longitudinal fileté (10) au droit dudit premier point de rupture (16) de façon à retirer une première portion (20) dudit corps longitudinal fileté (10) s'étendant dudit premier point de rupture (16) à ladite extrémité formant pointe (14), ou au droit dudit deuxième point de rupture (18) de façon à retirer une deuxième portion (26) dudit corps longitudinal fileté (10) s'étendant dudit deuxième point de rupture (18) à ladite extrémité formant pointe (14).

4. Vis de fixation selon l'une quelconque des revendications 1 à 3, **caractérisée en ce que** ladite tête de vis (12) forme une rotule dans laquelle est pratiqué un évidement (28), opposé audit corps longitudinal fileté (10), ledit évidement (28) étant susceptible de recevoir une clé pour entraîner en rotation ladite tête (12).

5. Vis de fixation selon l'une quelconque des revendications 1 à 4, **caractérisée en ce qu'**elle est réalisée en acier inoxydable.

6. Vis de fixation selon l'une quelconque des revendications 1 à 5, **caractérisée en ce qu'**elle est réalisée en alliage de titane.

7. Vis de fixation selon l'une quelconque des revendications 1 à 6, **caractérisée en ce qu'**une partie dudit corps longitudinal fileté s'étend entre ladite tête (12) et ledit au moins un point de rupture (16) le plus proche de la tête.

8. Ensemble de stabilisation du rachis, **caractérisé en ce qu'**il comprend au moins une vis de fixation selon l'une quelconque des revendications 1 à 7, ladite vis de fixation étant susceptible d'être vissée dans le pédicule d'une vertèbre.

## Claims

1. A fixing screw comprising a threaded longitudinal body (10) extending between a head (12) adapted to be driven in rotation and a point-forming end (14) suitable for being inserted into a support, which screw is **characterised in that** said threaded longitudinal body (10) includes:
- at least one break point (16) to enable said threaded longitudinal body (10) to be forcibly broken at said break point (16) in order to remove a portion (20) of said longitudinal body (10) extending from said break point (16) to said point-forming end (14), said break point (16) being formed by a groove at the periphery of said threaded longitudinal body (10), and
- at least one frustoconical portion flared towards the head (12) and extending from said break point towards the head (12) to constitute a new point-forming end after said portion (20) of said longitudinal body (10) has been removed.

2. A fixing screw according to claim 1, **characterised in that** the wall of the groove (22) nearer said head (12) forms said frustoconical portion which is flared towards said head (12) to constitute said new point-forming end (24) after said threaded longitudinal body (10) has been broken.

3. A fixing screw according to claim 1 or claim 2, **characterised in that** said threaded longitudinal body includes a first break point (16) and a second break point (18) between said head (12) and said first break point (16) so that said threaded longitudinal body (10) can be forcibly broken at said first break point (16) to remove a first portion (20) of said threaded longitudinal body (10) extending from said first break point (16) to said point-forming end (14) or at said second break point (18) to remove a second portion (26) of said threaded longitudinal body (10) extending from said second break point (18) to said point-forming end (14).

4. A fixing screw according to any one of claims 1 to 3, **characterised in that** said screw head (12) forms a ball in which there is formed a recess (28) facing away from said threaded longitudinal body (10), said recess (28) being adapted to receive a key to drive rotation of said head (12).

5. A fixing screw according to any one of claims 1 to 4, **characterised in that** it is made of stainless steel.

6. A fixing screw according to any one of claims 1 to 5, **characterised in that** it is made of titanium alloy.

7. A fixing screw according to any one of claims 1 to 6, **characterised in that** a portion of said threaded longitudinal body extends between said head (12) and said at least one break point (16) nearer the head.

8. A spine stabilizing system, **characterised in that** it includes at least one fixing screw according to any one of claims 1 to 7 adapted to be screwed into a pedicle of a vertebra.

## Patentansprüche

1. Befestigungsschraube mit einem Gewindelängskörper (10), der sich zwischen einem Kopf (12), der dazu bestimmt ist, diesen in Drehung zu versetzen, und einem eine Spitze bildenden Ende (14), das in eine Halterung eingeführt zu werden vermag, erstreckt,
**dadurch gekennzeichnet, dass** der Gewindelängskörper (10) umfasst:
- wenigstens eine Sollbruchstelle (16), um den Gewindelängskörper (10) unter Kraftaufwand rechtwinklig zu der Sollbruchstelle (16) durchbrechen zu können, um ein Stück (20) des Längskörpers (10), das sich von der Sollbruchstelle (16) bis zu dem die Spitze bildenden Ende (14) erstreckt, entfernen zu können, wobei die Sollbruchstelle (16) aus einer Nut gebildet ist, die im Umfang des Gewindelängskörpers (10) ausgebildet ist, und
- wenigstens ein kegelstumpfartiges, zum Kopf (12) hin erweitertes Stück, wobei das kegelstumpfartige Stück sich von der Sollbruchstelle zum Kopf (12) erstreckt, um nach dem Entfernen des Stücks (20) aus dem Längskörper (10) ein neues eine Spitze bildendes Ende zu bilden.

2. Befestigungsschraube nach Anspruch 1,
**dadurch gekennzeichnet, dass** die dem Kopf (12) am nächsten gelegene Wand der Nut (22) das kegelstumpfartige, zum Kopf (12) hin erweiterte Stück bildet, um nach Durchbrechen des Gewindelängskörpers (10) das neue eine Spitze bildende Ende (24) zu bilden.

3. Befestigungsschraube nach einem der Ansprüche 1 oder 2,
**dadurch gekennzeichnet, dass** der Gewindelängskörper eine erste Sollbruchstelle (16) und eine zwischen dem Kopf (12) und der ersten Sollbruchstelle (16) gelegene zweite Sollbruchstelle (18) umfasst, zum Durchbrechen des Gewindelängskörpers (10) unter Kraftaufwand rechtwinklig zu der ersten Sollbruchstelle (16), um ein erstes Stück (20) des Gewindelängskörpers (10) zu entfernen, das sich von der ersten Sollbruchstelle (16) bis zu dem die Spitze bildenden Ende (14) erstreckt, oder rechtwinklig zu der zweiten Sollbruchstelle (18), um ein zweites Stück (26) des Gewindelängskörpers (10) zu entfernen, das sich von der zweiten Sollbruchstelle (18) bis zu dem die Spitze bildenden Ende (14) erstreckt.

4. Befestigungsschraube nach einem der Ansprüche 1 bis 3,
**dadurch gekennzeichnet, dass** der Schraubenkopf (12) ein Kugelgelenk bildet, in das an der dem Gewindelängskörper (10) abgewandten Seite eine Aussparung (28) ausgebildet ist, wobei die Aussparung (28) einen Schlüssel aufzunehmen vermag, um den Kopf (12) in Drehung zu versetzen.

5. Befestigungsschraube nach einem der Ansprüche 1 bis 4,
**dadurch gekennzeichnet, dass** sie aus nicht rostendem Stahl ausgeführt ist.

6. Befestigungsschraube nach einem der Ansprüche 1 bis 5,
**dadurch gekennzeichnet, dass** sie aus Titan-Legierung ausgeführt ist.

7. Befestigungsschraube nach einem der Ansprüche 1 bis 6,
**dadurch gekennzeichnet, dass** ein Teil des Gewindelängskörpers sich zwischen dem Kopf (12) und wenigstens einer dem Kopf am nächsten gelegenen Sollbruchstelle (16) erstreckt.

8. Einheit zum Stabilisieren der Wirbelsäule,
**dadurch gekennzeichnet, dass** sie wenigstens eine Befestigungsschraube nach einem der Ansprüche 1 bis 7 umfasst, wobei die Befestigungsschraube in den Bogenfuß eines Wirbels eingeschraubt zu werden vermag.
